Europäisches Patentamt

European Patent Office

Office européen des brevets

(11) Publication number: **0 119 248**
**B1**

(12) ## EUROPEAN PATENT SPECIFICATION

(45) Date of publication of patent specification: **12.04.89**

(51) Int. Cl.⁴: **A 61 F 13/06**

(21) Application number: **83903066.5**

(22) Date of filing: **12.09.83**

(86) International application number:
**PCT/US83/01377**

(87) International publication number:
**WO 84/01102 29.03.84 Gazette 84/09**

(54) ANTI-BACTERIAL MEDICAL MATERIAL AND DEVICE.

(30) Priority: **13.09.82 US 416906**

(43) Date of publication of application:
**26.09.84 Bulletin 84/39**

(45) Publication of the grant of the patent:
**12.04.89 Bulletin 89/15**

(84) Designated Contracting States:
**BE DE FR GB LU NL**

(56) References cited:
**DE-A-3 131 073**
**US-A-2 326 159**
**US-A-3 482 569**
**US-A-3 598 127**
**US-A-3 699 956**
**US-A-3 848 603**
**US-A-3 949 742**
**US-A-4 186 745**
**US-A-4 310 509**
**US-A-4 381 380**

(73) Proprietor: **HYDROMER INC.**
**Salem Industrial Park United States Route 22**
**Post Office Box 337 Whitehouse NJ 08888 (US)**

(72) Inventor: **CREASY, Walter, S.**
**272 White Oak Ridge**
**Bridgewater, NJ 08807 (US)**
Inventor: **LORENZ, Donald, H.**
**12 Radel Place**
**Basking Ridge, NJ 07920 (US)**

(74) Representative: **von Füner, Alexander, Dr. et al**
**Patentanwälte v. Füner, Ebbinghaus, Finck**
**Mariahilfplatz 2 & 3**
**D-8000 München 90 (DE)**

Courier Press, Leamington Spa, England.

## Description

### Technical field

This invention is in the field of medical materials and devices.

### Background of the invention

It is known to react polyvinylpyrrolidone (N-vinyl-2-pyrrolidone) with an isocyanate prepolymer and cure the reaction product to form polyvinylpyrrolidone bonded to a polyurethane to form, for example, coatings having a very low coefficient of friction when wetted with an aqueous solution. Reference may be had to U.S. patents 4,100,309 and 4,119,094 which are incorporated herein by reference. Such material does not dissolve when exposed to water or body fluids. It is also known to form complexes of iodine with polyvinylpyrrolidone (see. U.S. patents 2,739,992 and 2,706,701. This material is water-soluble and would be washed away by body fluids.

In accordance with this invention an anti-bacterial agent which for some applications is also an anti-fungal agent such as iodine is complexed with polyvinylpyrrolidone which has been rendered insoluble in water and body fluids by being complexed with a polyurethane, thus rendering the polyvinylpyrrolidone insoluble while still permitting the release of the anti-bacterial agent. The thus-formed material is useful per se to supply an anti-bacterial agent in the form of a pad, a bandage or the like or as a coating for a medical device such as a catheter. The material may be used, for example, between the toes in the treatment of athlete's foot (tinia pedis) which involves a fungal infection by trychophyton mentagrophytes.

Iodine complexed with insoluble polyvinylpyrrolidone has been made by using special alkaline catalysts when polymerizing (see patent 3,136,755 incorporated herein by reference) but these complexes are not useful for coating medical devices because they are not soluble in any solvents and cannot be used as a coating and they do not form the useful sponge-like materials of this invention.

### Brief summary of the invention

An anti-bacterial medical material which may also have anti-fungal activity, comprises a polyurethane complexed with polyvinylpyrrolidone and an anti-bacterial agent (which may also have anti-fungal activity) complexed with the polyvinylpyrrolidone. The material is used per se and as a coating for medical devices such as, for example, catheters and wound drainage tubes.

### Brief description of the drawings

Figure 1 is a front elevation of a device for treating athlete's foot.

Figure 2 is a rear elevation of the device of Figure 1.

Figure 3 is a right-side elevational view of the device of Figure 1.

Figure 4 is a plan view, partially broken away, showing the device of Figure 1 between two toes.

Figure 5 is a plan view, partially broken away, of a peristaltic pump tube.

Figure 6 is a plan view of a urethral catheter, partially broken away.

### Detailed description

The medical material in accordance with the invention comprises a polyurethane complexed with polyvinylpyrrolidone which in turn is complexed with an anti-bacterial agent which may also be an anti-fungal agent.

It is known to complex a polyurethane with polyvinylpyrrolidone. The exact nature of the complexing is not known but the polyvinylpyrrolidone is, in any event bound to the polyurethane in a form which is referred to in the art as a complex (some refer to this particular complex as an interpolymer). These complexes and the manner of making them are well-known to the art as seen, for example, in U.S. patent 4,100,309.

Typical polyvinylpyrrolidone-polyurethanes complexes are polyvinylpyrrolidone complexed with polytetramethylene ether glycol-diphenylmethane diisocyanate (MDI), polytetramethylene ether glycol-tolylene diisocyanate (TDI), polytetramethylene ether glycolisoferrone isocyanate, poly(1,4-oxybutylene) glycoldiphenylmethane diisocyanate (MDI), poly(1,4-oxybutylene) glycol-tolylene diisocyanate (TDI), poly(1,4-oxybutylene) glycol-isoferrone isocyanate, polyethylene glycol-diphenylmethane diisocyanate (MDI), polyethylene glycoltolylene diisocyanate (TDI), polyethylene glycolisoferrone isocyanate, polypropylene glycol-diphenylmethane diisocyanate (MDI), polypropylene glycol-tolylene diisocyanate (TDI), polypropylene glycol-isoferrone isocyanate, polycaprolactone-diphenyl-methane diisocyanate (MDI), polycaprolactone-tolylene diisocyanate (TDI), polycaprolactone-isoferrone isocyanate, polyethylene adipate-diphenylmethane diisocyanate (MDI), polyethylene adipate-tolylene diisocyanate (TDI), polyethylene adipate-isoferrone isocyanate, polytetramethylene adipate-diphenylmethane diisocyanate (MDI), polytetramethylene adipate-tolylene diisocyanate (TDI), polytetramethylene adipate-isoferrone isocyanate, polyethylene-propylene adipate-diphenylmethane diisocyanate (MDI), polyethylene-propylene adipate-tolylene diisocyanate (TDI), or polyethylene-propylene adipate-isoferrone isocyanate polyurethanes.

Preferred prepolymers for use in making the material of the invention are pentaerythritalethoxylatetoluene diisocyanate prepolymer and methylene bis phenylisocyanate (MDI)-rincinoleraic acid glyceride prepolymer. Suitable polyurethane foam prepolymers are disclosed in U.S. patent 4,137,200 and 4,160,076. Reference may be had to Encyclopedia of Polymer Science and Technology, H. F. Mark, N. G. Gaylord, and N. M. Bakels (Eds.) (1969), incorporated herein by reference, for further polyurethanes and prepolymers for making polyurethanes.

When the material of the invention is to be used per se as distinguished from being used as a coating on a substrate, it is preferred to use a resilient open-celled hydrophilic polyurethane foam. Advantageously the foam will have a density of from about 1.5 to about 15 lbs. Such polyurethane foams are well-known to the art. Exemplary are the above polyurethanes which are blown on being produced using water. Such polyurethane foams are disclosed in U.S. patents 4,160,076 and 4,137,200 which are incorporated herein by reference.

The material of the invention can be made either from pre-formed polyurethane which is then reacted with an isocyanate prepolymer and polyvinylpyrrolidone to bind the polyvinylpyrrolidone to the polyurethane or the polyvinylpyrrolidone can be mixed with an isocyanate prepolymer before it is used to form a polyurethane foam.

When the material of the invention is to be used as a coating on a substrate, a polyisocyanate prepolymer and polyvinylpyrrolidone are placed in a solvent and the thus-formed solution is applied to the substrate by dipping, spraying, brushing or the like. Typically, the thus-formed coating is air-dried and then cured at, for example, 60°C. for about 15 minutes. Typical substrates are polyurethanes, vinyl resins such as polyvinylchloride, polyacrylates such as polymethylmethacrylate, polycarbonates, polyesters such as polyethylene terephthalate, polybutylene terephthalate, polytetramethyl terephthalate or rubber such as latex rubber or polyisoprene.

Advantageously, the polyvinylpyrrolidone will have a K-value of at least 85. Preferably, the polyvinylpyrrolidone bound to the polyurethane will be from about 1% to about 20%, preferably from about 5% to about 15%, by weight of the combined weight of the polyurethane and the polyvinylpyrrolidone.

The anti-bacterial agent may be, for example, a halide such as a triiodide, for example, potassium triiodide; a halogen, for example, iodine and chlorine, hexachlorophene, griseofulvin or miconazole nitrate. The anti-bacterial agent is complexed with the polyvinylpyrrolidone by immersing the cured polyurethanepolyvinylpyrrolidone complex in a solution of the anti-bacterial agent using a solvent which is not a solvent for the polyvinylpyrrolidone-urethane complex, i.e. a water, in the case of potassium triiodide. This agent will be present in widely varying amounts depending on the application but generally will be present in an amount of from about 1% to about 20% by weight of the polyvinylpyrrolidone-polyurethane complex.

Example 1

A slab of conventional resilient open-celled polyurethane (water-foamed polyethylene glycol-trimethylolpropane-toluene diisocyanate) of density of about 2 was dipped into a solution containing the following ingredients: 300 ml of methyl ethyl ketone, 100 ml of ethyl lactate, 9 gm of polyvinylpyrrolidone (K-90) and 2.25 gm of an isocyanate terminated prepolymer (methylenebis phenyl isocyanate (MDI) and ricinoleraic acid glyceride). Excess solution was removed by running the foam through a squeezer roll. The foam was then air-dried for 30 minutes and finally heated in a forced draft oven at 60°C. for 1 hour. The foam was removed, cooled to room temperature and washed with water to remove any unreacted polyvinylpyrrolidone. Excess water was removed by again running the foam through a squeezer roll. The damp foam was then dipped into a 0.01N potassium triiodide water solution for 1 minute. The excess solution was removed by running the foam through a squeezer roll and the foam was washed with water until no iodine color was observed in the wash water—about 2 washes. The foam was then dried in a forced draft oven at 60°C. for 3 hours. The available iodine in the foam was determined by taking a weighted sample of foam and adding it to a standard sodium thiosulfate solution and agitating for 24 hours and finally back titrating the solution with standard triiodide solution using a starch indicator solution. Under these conditions the foam contains approximately 6% available iodine.

Using standard microbiological tests, pieces of the foam showed significant zones of inhibition against staph aureus, candida albicans, and trichophyton mentagrophytes, using the spread plate method. Using the AOAC Method to determine sporocidal activity, kill was obtained at 10 minutes using a sample of the foam. Controls of the untreated foam itself and also the foam was bound polyvinylpyrrolidone without complexed iodine showed no activity.

Example 2

A hydrophilic polyurethane foam bun was prepared in the following manner. Polyvinylpyrrolidone (K-90) 100 gms was dissolved in 900 gms of water to which was added 8.0 gm of a surface active agent having an HLB of 13.5 (Triton X-100) and 2.0 gm of a liquid defoaming agent having an HLB of 7 (Pluronic L-62 of BASF Wyandotte). The aqueous solution was mixed with 1000 gms of Hypol 2002 (an isocyanate terminated prepolymer made of toluene diisocyanate and an ethoxylated glycerine, see U.S. patent 4,160,076) and quickly poured into a pan. After the foam had risen it was allowed to stand for 3 days to finish curing. It was then sliced and pieces were punched out which were of a biconcave structure with anvil-shaped ends. They were washed with water to remove any unreacted polyvinylpyrrolidone and finally reacted with a potassium triiodide water solution (0.01N) for 1 minute with agitation. The foam pieces were then washed with water to remove any excess triiodide not complexed to the polyvinylpyrrolidone and dried in an oven at 60°C. for 1 hour. The available iodine was determined as in Example 1 to be 5.5%.

Microbiological tests confirmed that the foam had killing power for staph aureus, candida albicans and trichophyton mentagrophytes.

Example 3

A polyvinylchloride urethral catheter was coated by dipping in a solution comprised of methyl ethyl ketone and 25 ml of diacetone alcohol containing 2.50 gm of polyvinylpyrrolidone (K-90) and 1.25 gm of pentaerytrital ethoxylate-toluene diisocyanate prepolymer (Hypol FHP3000 of W. R. Grace). The coating was air-dried for 5 minutes and cured at 60°C. for 15 minutes. The coated catheter was then wetted with water and then placed in a 0.01 N potassium triiodide solution for 1 minute. The coating immediately picked up the iodine color. After drying the iodine-containing coating and washing with fresh distilled water it was observed that the iodine color remained even on heating at 60°C. for 24 hours. Titration of the available iodine with sodium thiosulfate confirmed the presence of active iodine.

A 1 cm length of the coated catheter was left in contact with a growing staph aureus colony at 37°C. for 15 minutes and then an aliquot was put on a culture plate. No growth was observed; a control without contact had 910 counts and one with contact with a non-iodinated coated tube has 900 counts. A similar experiment using candida albicans again showed complete kill. Similar experiments of higher levels of available iodine showed it also killed trichophyton mentagrophytes.

Example 4

The coating of a urethane tube is accomplished by dipping in a solution containing 300 ml of methyl ethyl ketone, 100 ml of ethyl lactate, 9 gm of polyvinylpyrrolidone (K-90) and 2.25 gm of isocyanate terminated ricinoleate prepolymer mixed with a chlorinated rubber as described in U.S. patent 4,217,254. After air drying for 5 minutes, the coating is cured at 60°C. for 1 hour. The coated tube is wetted with water and then dipped in 0.1 N potassium triiodide solution for 30 seconds. The coating rapidly picks up the iodine color of the solution and retains it both on washing with water and on heating. The iodine content is titrable with sodium thiosulfate showing it has available iodine.

Similar experiments as in Example 3 shows it to be bactericidal, and that it kills fungi and yeasts.

Example 5

A coating on a polyvinylchloride sheet was prepared, following the procedure of Example 4, but the wetted coating was placed in a 0.1% solution of hexachlorophene in water for 1 hour. The concentration of the hexachlorophene after being in contact was measured and it was found to have decreased due to the complexation with the coating.

The material of the invention can be used, for example, as a device for delivering an anti-bacterial agent and/or an anti-fungal agent to a body site. Advantageously the material will be in the form of a pad. It is preferred to have it in the form of a foam pad.

A typical device is a pad formed of a complexed polyurethane-polyvinylpyrrolidone with the polyvinylpyrrolidone complexed with an agent which has both anti-fungal and anti-bacterial activity for the treatment of athlete's foot. Advantageously the pad is made of a foam formed using a foamed polyurethane, for example, a hydrophilic foamed polyurethane formed from a cross-linked isocyanate-capped polyoxethylene polyol prepolymer. Examples of suitable cross-linked foams are found in patents 4,137,200 and 4,160,076. The polyvinylpyrrolidone may be complexed with the foamed polyurethane either by mixing it with the prepolymer or after the polyurethane foam is formed as detailed above.

The work of Examples 1, 2, 3 and 5 was actually carried out.

The preferred agents are iodine, potassium triiodide, or miconazole nitrate and hexachlorophene which are complexed with the polyvinylpyrrolidone as detailed above.

The pad will be sized to fit between two toes and have a height about equal to the thickness (depth) of the toes. The sides of the pad are preferably concave to accommodate the toes.

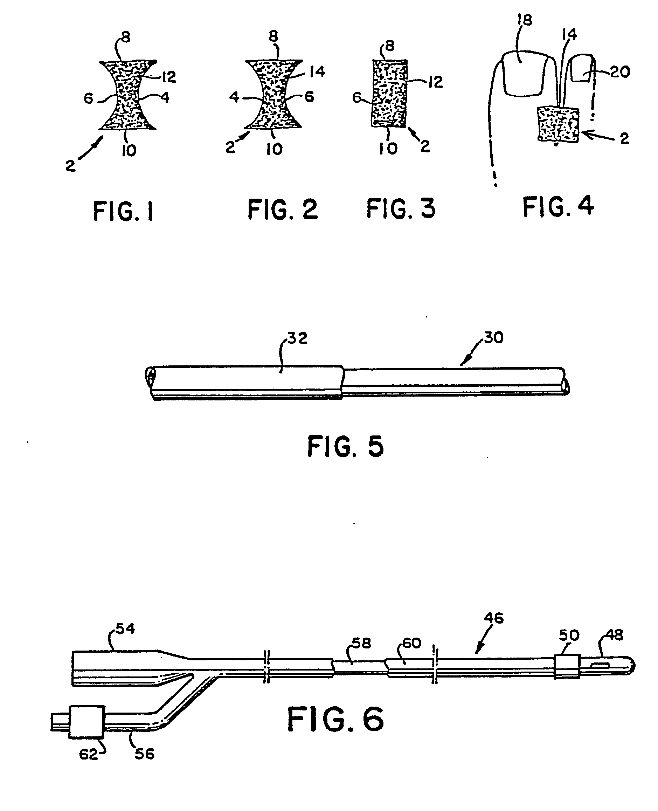

Referring to Figure 1, a polyurethane sponge 2 has concave sides 4 and 6, a flat top 8 and a flat bottom 10. It also has a substantially flat front 12 and a substantially flat rear 14. The concave sides 4 and 6 provide for a comfortable fit of the sponge 2 between a pair of toes such as a big toe 18 and an adjacent toe 20. The concave sides 4 and 6 also assist in retaining the sponge 2 between said toes.

Sponge 2 may be formed of the material made in example 2, thus providing for the gradual release of iodine for the treatment of athlete's foot on toes 18 and 20.

Referring now to Figure 5, a peristaltic pump tube 30 of polyvinylchloride has a coating 32 of a complexed polyurethane-polyvinylpyrrolidone having the polyvinylpyrrolidone complexed with potassium triiodide. This tube can be formed following the general procedure outlined in example 3.

Referring to Figure 6, a urethral catheter 46 has a tip 48, a balloon portion 50, a drain connector 54 and a valve branch 56, 62 formed from a branched tube 58 of rubber latex coated (60) with cross-linked polyurethane-polyvinylpyrrolidone with the polyvinylpyrrolidone complexed with hexachlorophene.

Claims

1. An anti-bacterial medical material comprising:

a polyurethane complexed with polyvinylpyrrolidone, and

an anti-bacterial agent complexed with said polyvinylpyrrolidone.

2. An anti-bacterial medical material in accordance with Claim 1 in which the anti-bacterial agent is iodine, an iodide, hexachlorophene, griseofulvin, or miconazole nitrate.

3. A material in accordance with Claim 1 or 2 in which the polyurethane is foamed.

4. An anti-bacterial article comprising:
a substrate (30, 58),
a coating (32, 60) on the substrate comprising a polyurethane complexed with polyvinylpyrrolidone and an anti-bacterial agent complexed with said polyvinylpyrrolidone.

5. An anti-bacterial article in accordance with Claim 4 in which the substrate is polyurethane, a vinyl resin, a polyacrylate, a polycarbonate, a polyester or rubber.

6. A device for treating athlete's foot comprising a pad (2) of a polyurethane foam complexed with polyvinylpyrrolidone which is complexed with an anti-bacterial and anti-fungal agent.

7. The device of Claim 6 in which the agent is iodine.

8. The device of Claim 6 in which the agent is an iodide.

9. The device of Claim 6 in which the agent is a halide, a halogen, hexachlorophene, griseofulvin or miconazole nitrate.

10. The device of any of Claims 6 through 9 in which the pad (2) has opposed concave sides (4, 6) for the reception of toes (18, 20).

**Patentansprüche**

1. Antibakteriell wirkender medizinischer Stoff aus einem Polyurethan-Polyvinylpyrrolidon-Komplex und einem mit dem Polyvinylpyrrolidon einen Komplex bildenden antibakteriellen Mittel.

2. Antibakteriell wirkender medizinischer Stoff nach Anspruch 1, worin das antibakterielle Mittel Jod, Jodid, Hexachlorophen, Griseofulvin oder Miconazolnitrat ist.

3. Stoff nach Anspruch 1 oder 3, worin das Polyurethan geschäumt ist.

4. Antibakterieller Gegenstand aus einem Substrat (30, 58) und einem Überzug (32, 60) auf dem Substrat, wobei der Überzug zusammengesetzt ist aus einem Polyurethan-Polyvinylpyrrolidon-Komplex und einem mit dem Polyvinylpyrrolidon einen Komplex bildenden antibakteriellen Mittel.

5. Antibakterieller Gegenstand nach Anspruch 4, worin das Substrat ein Polyurethan, ein Vinylharz, ein Polyacrylat, ein Polycarbonat, ein Polyester oder Kautschuk ist.

6. Vorrichtung zur Behandlung von Fußpilz aus einem Tampon (2) aus mit Polyvinylpyrrolidon einen Komplex bildendem Polyurethanschaum, wobei das Polyvinylpyrrolidon wiederum mit einem antibakteriellen und fungiziden Mittel zu einem Komplex verbunden ist.

7. Vorrichtung nach Anspruch 6, wobei das Mittel Jod ist.

8. Vorrichtung nach Anspruch 6, wobei das Mittel Jodid ist.

9. Vorrichtung nach Anspruch 6, wobei das Mittel ein Halogenid, ein Halogen, Hexachlorphen, Griseofulvin oder Micronazolnitrat ist.

10. Vorrichtung nach einem der Ansprüche 6 bis 9, wobei der Tampon (2) gegenüberliegende konkave Seiten (4, 6) für die Aufnahme der Zehen (18, 20) aufweist.

**Revendications**

1. Produit médical bactéricide, comprenant:
—un complexe de polyuréthane avec un polyvinylpyrrolidone, et
—un agent bactéricide complexé avec le polyvinylpyrrolidone.

2. Produit médical bactéricide selon la revendication 1, dans lequel l'agent bactéricide est l'iode, un iodure, l'hexachlorophène, la griséofulvine, ou un nitrate de miconazole.

3. Produit selon la revendication 1 ou la revendication 2, dans lequel le polyuréthane est formé en mousse.

4. Objet bactéricide comprenant:
—un substrat (30, 58), et
—un revêtement (32, 60) sur le substrat, comprenant le complexe de polyuréthane et de polyvinylpyrrolidone, et un agent bactéricide complexé avec le polyvinylpyrrolidone.

5. Objet bactéricide selon la revendication 4, dans lequel le substrat est un polyuréthane, une résine vinylique, un polyacrylate, un polycarbonate, un polyester ou du caoutchouc.

6. Dispositif pour traiter le pied d'athlète, comprenant um tampon (2) d'une mousse de polyuréthane formant un complexe avec un polyvinylpyrrolidone, lequel est lui-même complexé avec un agent bactéricide et fongicide.

7. Dispositif selon la revendication 6, dans lequel l'agent est de l'iode.

8. Dispositif selon la revendication 6, dans lequel l'agent est un iodure.

9. Dispositif selon la revendication 6, dans lequel l'agent est un halogénure, un halogène, l'hexachlorophène, la griséofulvine ou le nitrate de micronazole.

10. Dispositif selon l'une quelconque des revendications 6 à 9, dans lequel le tampon (2) a des côtés opposés concaves (4, 6) pour recevoir des orteils (18, 20).

FIG. 1

FIG. 2

FIG. 3

FIG. 4

FIG. 5

FIG. 6